Europäisches Patentamt

European Patent Office

Office européen des brevets

(11) Numéro de publication: **0 514 665 B1**

# FASCICULE DE BREVET EUROPEEN

(45) Date de publication de fascicule du brevet: **18.01.95** (51) Int. Cl.⁶: **A61K 7/00**, A23D 9/06, C11B 5/00

(21) Numéro de dépôt: **92106468.9**

(22) Date de dépôt: **15.04.92**

(54) **Mélange antioxydant liposoluble.**

(30) Priorité: **24.05.91 EP 91108412**

(43) Date de publication de la demande: **25.11.92 Bulletin 92/48**

(45) Mention de la délivrance du brevet: **18.01.95 Bulletin 95/03**

(84) Etats contractants désignés: **AT BE CH DE DK ES FR GB IT LI NL SE**

(56) Documents cités:
**EP-A- 0 326 829
EP-A- 0 353 161
GB-A- 902 377
US-A- 2 432 698**

**REVUE FRANCAISE DES CORPS GRAS vol. 34, no. 5,6, Mai 1987, PARIS, FR pages 271**

**- 274; CILLARD J. ET AL: 'Antioxidant activity of associated alpha-tocopheroland ascorbic acid in aqueous media.'**

(73) Titulaire: **SOCIETE DES PRODUITS NESTLE S.A.
Case postale 353
CH-1800 Vevey (CH)**

(72) Inventeur: **Colarow, Ladislas
Eden-Roc 12
CH-1073 Savigny (CH)**

**Description**

L'invention concerne un mélange antioxydant liposoluble à base d'acide organique.

L'intérêt qu'il y a à présenter les acides organiques antioxydants sous une forme liposoluble est dû au fait qu'ils exercent une activité inhibitrice de l'oxydation des lipides dans les systèmes biologiques. En combinaison avec la vitamine E, la vitamine C par exemple exerce une acitvité antioxydante synergique car, en tant que réducteur, l'acide L-ascorbique transforme les dérivés tocophéroxyles en tocophérols qui peuvent à nouveau fixer les radicaux libres sous forme de dérivés tocophéroxyles.

L'acide L-ascorbique, hydrophile, peut être rendu liposoluble par estérification de ses groupes hydroxyles en position 5 ou 6 avec un acide gras. Ainsi, l'acide 6-0-palmitoyl-L-ascorbique ou ascorbyl-palmitate est utilisé comme additif pour protéger les matières grasses de l'oxydation et également comme source de vitamine C. Ce composé présente cependant l'inconvénient de se dissoudre extrêmement lentement dans les matières grasses aux températures auxquelles il ou elles ne se dégradent pas. On a ainsi recours à l'addition de glycérides partiels ou de lipides complexes, par exemple de lécithine de soja. Mais même avec ces additifs, l'incorporation de palmitate d'ascorbyle à un niveau assurant un pouvoir antioxydant suffisant ne peut s'effectuer qu'à température relativement élevée, de l'ordre de 130°C pendant une certaine durée, par exemple 30 min. ce qui induit une dégradation des matières grasses comme de l'antioxydant lui-même dont le point de fusion est 115,5°C.

On a proposé, par exemple selon la demande de brevet EP-A-0326829 de réaliser un mélange antioxydant liposoluble solide à température ambiante contenant l'acide ascorbique à base de lipides polaires tels que les lécithines. Un tel mélange peut être utilisé sans inconvénient dans les produits solides secs.

Son incorporation dans les huiles riches en acides gras polyinsaturés sensibles à la chaleur nécessite cependant l'intervention d'un solvant polaire, par exemple l'éthanol qui doit ensuite être éliminé.

De plus, son incorporation dans des produits contenant de l'eau, par exemple des émulsions, est problématique car l'acide ascorbique en présence d'eau a tendance à provoquer un dégommage de la lécithine, ce qui a une influence négative sur leur stabilité.

Par ailleurs, selon US-A-2.432.698 par exemple, les vitamines A et D peuvent être protégées de l'oxydation par addition de niacinamide. Lorsqu'il s'agit de stabiliser ces vitamines avec comme support une huile, le cas échéant en présence de tocophérol, la niacinamide n'étant pas liposoluble, on a recours selon ce brevet à l'addition au mélange de solvants naturels comme par exemple les alcools éthylique et laurique.

L'invention se propose de résoudre le problème de l'incorporation d'acides organiques, notamment de l'acide ascorbique dans un mélange antioxydant liposoluble constitué d'ingrédients naturels ne présentant pas les inconvénients précités et pouvant être incorporé dans des produits anhydres comme dans des produits humides, par exemple dans des émulsions.

Le mélange antioxydant selon l'invention est caractérisé par le fait qu'il est constitué d'un complexe de vitamine du groupe B et d'acide organique et d'une lécithine contenant au moins 40% en poids de phosphatidylcholine.

Parmi les vitamines du groupe B, on préfère les bases pyridiniques, par exemple la niacinamide, la pyridoxine ou la pyridoxamine, la niacinamide étant préférée.

Par acide organique selon l'invention, on entend un polyacide capable de former un complexe avec une base vitaminique du groupe B, tel que par l'exemple l'acide citrique, tartrique ou de préférence l'acide ascorbique.

Dans le contexte de l'invention, la lécithine utilisée est de préférence une fraction de lécithine de soja soluble dans l'alcool éthylique. Celle-ci est zwitterionique c'est-à-dire stable dans une gamme large de pH et contient peu de phospholipides complexables. La lécithine peut se présenter de préférence sous forme d'un mélange contenant environ 40-50% en poids de phosphatidylcholine et environ 50-60% en poids d'huile végétale, par exemple de soja, de triglycéride à chaîne moyenne (triglycéride d'acides gras en C8-C10) ou de beurre de cacao.

Pour préparer le mélange antioxydant liposoluble, on dissout le complexe d'acide organique et de vitamine du groupe B, par exemple l'ascorbate de niacinamide, de préférence dans un solvant de qualité alimentaire, par exemple l'éthanol ayant été de préférence préalablement dégasé. Le complexe lui-même peut être préparé, par exemple par chauffage modéré d'un mélange équimolaire de niacinamide et d'acide ascorbique dans un solvant polaire approprié, par exemple l'alcool éthylique, l'acétone ou l'eau. La dissolution du complexe peut s'effectuer par agitation modérée à température de 40 -50°C, de préférence sous atmosphère inerte, par exemple d'azote, à une concentration de 2 à 10 g de complexe pour 100 ml de solvant. On y ajoute ensuite le cas échéant le d,l-alphatocophérol.

En variante, on peut dissoudre directement la vitamine du groupe B, l'acide organique et la vitamine E dans le solvant dans les mêmes conditions que précédemment, pourvu que la vitamine du groupe B et l'acide organique soient en proportions équimolaires de manière que le complexe puisse se former.

5-10 volumes de cette solution sont alors mélangés à 1-2 volumes de lécithine qui se dissout rapidement sous agitation modérée à 50-60°C, de préférence sous atmosphère inerte. On peut ensuite concentrer la solution en évacuant le solvant, par exemple par distillation sous gaz inerte à 60-65°C, puis éliminer toute trace de solvant résiduel, par exemple par barbottage d'un gaz inerte à la température ambiante. Le mélange obtenu se présente sous forme d'une solution translucide de couleur jaune clair semblable à une huile végétale raffinée ayant la viscosité d'une lécithine de soja standard du commerce, c'est-à-dire contenant environ 35-40% en poids d'huile de soja. Il a une coloration ambrée, est translucide, inodore et stable à la chaleur.

Le mélange antioxydant peut être entreposé à l'abri de la lumière et à température ambiante.

Il peut être utilisé tel quel et être facilement incorporé dans une huile destinée à être protégée de l'oxydation, par exemple une huile riche en acides gras insaturés, de préférence à raison de 1 à 2% en poids.

Dans un mode de réalisation préféré, le mélange antioxydant peut se présenter sous une forme plus fluide et contenir par exemple un triglycéride à chaîne moyenne.

Selon une mise en oeuvre particulièrement avantageuse du point de vue de l'activité antioxydante, le mélange contient en outre du tocophérol procurant une synergie. Comme tocophérol, on peut utiliser l'alpha-, le bêta-, le gamma-, le delta-tocophérol ou un mélange de ces tocophérols, par exemple un mélange naturel provenant de la fraction insaponifiable d'une huile végétale, par exemple de l'huile de soja, de l'huile de germes de blé, de l'huile de graines de coton. Le tocophérol peut être ajouté au mélange antioxydant ou se trouver naturellement présent dans l'huile destinée à être protégée.

Le mélange antioxydant contient avantageusement 1 à 5%, de préférence environ 2,5% en poids de tocophérol et 2,5 à 30%, de préférence 5 à 20% en poids de complexe, par exemple d'ascorbate de niacinamide.

Le mélange antioxydant peut servir de support à d'autres vitamines liposolubles, par exemple la vitamine A et constituer un micronutriment de grande valeur nutritionnelle lorsqu'il contient par exemple du MCT.

Dans le même ordre d'idées, il peut servir de support vitaminique destiné à être incorporé dans la phase lipidique lors de la préparation d'un aliment diététique tel que, par exemple un lait infantile et constituer alors un moyen particulièrement avantageux d'introduction des vitamines B et C, non liposolubles.

Le mélange antioxydant peut être incorporé dans un produit alimentaire contenant une matière grasse insaturée, particulièrement une huile végétale, par exemple de germes de blé, de pépins de cassis, de Kiwi, de maïs, de soja, de carthame, d'olive, d'onagre, de bourrache ou une matière grasse animale comme l'huile de beurre, la graisse de poule et particulièrement une huile de poisson.

Ce peut être par exemple une sauce à salade ou un produit diététique.

Le mélange antioxydant selon l'invention du fait de sa faible acidité, de pH ≧ 4, peut être aisément encapsulé, par exemple en gélules sans que l'eau résiduelle associée à la gélatine ne conduise au dégommage de la lécithine lors d'un entreposage prolongé.

Le mélange antioxydant peut être utilisé pour la protection des lipides contre l'oxydation dans les compositions destinées à l'alimentation entérale et parentérale.

Le mélange antioxydant peut également servir à la protection contre l'oxydation des lipides des compositions cosmétiques.

Les exemples ci-après illustrent l'invention. Dans ces exemples les parties et pourcentages sont pondéraux sauf indication contraire.

Exemple 1

On dissout 96,7 g d'ascorbate de niacinamide dans 5 l d'éthanol ayant été préalablement dégasé par sonication pour en éliminer l'oxygène libre, sous agitation modérée à 50°C sous un courant d'azote, puis on y ajoute 28,1 g de d,l-alpha-tocophérol. On pompe ensuite cette solution dans un flacon évaporateur rotatif de 25 l dans lequel on a préalablement introduit 1000 g de lécithine contenant 45% de phosphatidylcholine de soja dissout dans l'huile de carthame, à 50°C, ce qui conduit à une rapide dissolution de la lécithine, cette opération étant effectuée sous un courant d'azote. On obtient ainsi une solution translucide dépourvue de matières insolubles. On concentre ensuite la solution jusqu'à évaporation complète du solvant à 60°C sous un faible courant d'azote, puis après avoir refroidi la solution à la température ambiante, on en élimine toute trace de solvant résiduel par barbottage d'azote. Le mélange antioxydant liposoluble obtenu a une coloration jaune pâle et une viscosité semblable à celle d'une lécithine commerciale courante.

Il a la composition suivante:

|  | % |
|---|---|
| d,l-alpha-tocophérol | 2,5 |
| Vitamine C (sous forme d'ascorbate de niacinamide) | 5 |
| Vitamine B3 (sous forme d'ascorbate de niacinamide) | 3,6 |
| Phosphatidylcholine | 40 |
| Huile de carthame | complément à 100 |

Exemple 2

Pour préparer un mélange antioxydant liposoluble plus fluide que celui de l'exemple 1, on procède comme à l'exemple 1 en ajoutant 2000 g d'une lécithine constituée de 45% de phosphatidylcholine, de 50% de triglycéride à chaîne moyenne (en C8-C10) et de 5% d'autres phosphatides.

Exemple 3

On prépare un mélange antioxydant liposoluble comme à l'exemple 1 sauf que la lécithine mise en oeuvre contient 45% de phosphatidylcholine, 50% de beurre de cacao et 5% d'autres phosphatides.

Exemple 4

On prépare un mélange antioxydant liposoluble comme à l'exemple 1, sauf que la lécithine mise en oeuvre contient 45% de phosphatidylcholine, 50% d'huile de soja et 5% d'autres phosphatides.

Exemple 5

On prépare un mélange antioxydant liposoluble comme à l'exemple 1, sauf que la lécithine mise en oeuvre contient 45% de phosphatidylcholine, 50% de triglycéride à chaîne moyenne (en C8-C10) et 5% d'autres phosphatides.

Exemple 6

On prépare un mélange antioxydant liposoluble comme à l'exemple 1, sauf que l'on n'y ajoute pas de d,l-alphatocophérol.

Exemple 7

On dissout 190 g de niacinamide, 270 g d'acide ascorbique, 130 g de d,l-alpha-tocophérol et 9,41 kg de lécithine (contenant 45-50 % de phosphatidylcholine pour 50-55 % de triglycérides à chaîne moyenne) dans 8 l d'éthanol de qualité alimentaire (à 94 %) à 50 °C dans un évaporateur en verre de 5O l jusqu'à l'obtention d'une solution claire, sous légère agitation dans un courant d'azote. On concentre ensuite la solution sous un vide de 250-60 mb à 60-70 °C jusqu'à dessiccation. Le produit obtenu est fluide à 50 °C et se dissout rapidement dans les huiles et les graisses à 40-60 °C.

Exemple 8

On dissout 4,49 g de pyridoxine, 4,67 g d'acide ascorbique et 2,37 g de d,l-alpha-tocophérol dans l'éthanol (à 94 %) à 50 °C, on ajoute à la solution 188,47 g de lécithine (contenant 45-50 % de phosphatidylcholine pour 50-55 % de triglycérides à chaîne moyenne), puis on concentre la solution jusqu'à dessiccation comme à l'exemple 7. Le produit obtenu est fluide et se dissout rapidement dans les huiles et les graisses à 40-60 °C.

Exemple 9

En procédant comme à l'exemple 7, on dissout 2,74 g de monohydrate d'acide citrique, 4,77 g de niacinamide, 2,5 g de d,l-alpha-tocophérol et 190 g de lécithine (contenant 45-50 % de phosphatidylcholine et 50-55 % de triglycérides à chaîne moyenne) dans 250 ml d'éthanol (à 94 %), puis on concentre la solution jusqu'à dessiccation. Le produit obtenu est fluide et se dissout rapidement dans les huiles et les graisses à 40-60°C.

Exemple 10

En procédant comme à l'exemple 7, on dissout 7,07 g de pyridoxine, 2,93 g de monohydrate d'acide citrique, 2,67 g de d,l-alpha-tocophérol et 183,33 g de lécithine (contenant 45-50 % de phosphatidylcholine et 50-55 % de triglycédides à chaîne moyenne) dans 250 ml d'éthanol (à 94 %), puis on concentre la solution jusqu'à dessiccation. Le produit obtenu est fluide à 50°C et se dissout rapidement dans les huiles à cette température.

Exemples 11-13

On prépare les huiles spéciales ci-après par simple dissolution, sous faible agitation à 40-50°C sous azote, du mélange antioxydant selon l'exemple 1 mais ne contenant pas de d,l-alpha-tocophérol dans une huile, puis on ajoute à la solution du d,l-alpha-tocophérol dans les proportions indiquées au tableau 1 ci-après:

Tableau I

| Composition (g) | Exemples | | |
|---|---|---|---|
| | 11 | 12 | 13 |
| Huile de kiwi riche en acides gras hautement insaturés | --- | --- | 1'000 |
| Huile de pépins de cassis | 1000 | --- | --- |
| Huile de poissons, riche en acide eicosapentaénoïque | --- | 1000 | --- |
| d,l-Alpha-tocophérol | 0,25 | 0,25 | 0,236 |
| Mélange antioxydant fournissant | 10,6 | 15,9 | 10 |
| ... g d'ascorbate de niacinamide | 0,5 | 0,75 | 0,472 |

Exemple 14

On prépare des capsules de gélatine, contenant 500 mg du mélange selon l'exemple 1 et les micronutriments indiqués dans les proportions indiquées. La préparation nutritive vitaminée a la composition indiquée ci-après:

| Composition | mg |
|---|---|
| Huile de pépins de cassis | 280 |
| Acétate de vitamine A (dissout dans l'huile de pépins de cassis) | 0,12 |
| Vitamine E sous forme de d,l-alpha-tocophérol (apporté par le mélange antioxydant selon l'exemple 1) | 1,33 |
| Tétrabutyrate de vitamine B2 (dissout dans l'huile de pépins de cassis) | 0,3 |
| Vitamine C (sous forme d'ascorbate de niacinamide et lécithine apporté par le mélange antioxydant selon l'exemple 1) | 2,661 |
| Vitamine B3 (sous forme d'ascorbate de niacinamide et lécithine apporté par le mélange antioxydant selon l'exemple 1) | 1,849 |
| Phosphatidylcholine de soja (sous forme de phosphatidylcholine dans l'huile de carthame) | 125 |
| Huile de carthame | 125 |

Les gélules sont stables à l'entreposage prolongé à la température ambiante.

Exemple 15

On prépare une huile destinée à assaisonner une salade de légumes frais sans vinaigre ayant la composition suivante.

Composition                                                                   g

────────────────────────────────────────────────────────────

Huile de carthame raffinée contenant          900
500 mg de d,l-alpha-tocophérol

Mélange antioxydant selon l'exemple 1          100
apportant 5 g de vitamine C
          3,6 g de vitamine B3 et
          40 g de phosphatidylcholine

Une assiette de légumes frais de goût neutre peut être agrémentée avec 10 g du mélange précédent et apporter ainsi 50 mg de vitamine C, 36 mg de vitamine B3 et 400 mg de phosphatidylcholine.

Exemple 16

En utilisant le test d'oxydation accéléré Rancimat[R], on détermine les temps d'induction de l'huile de pépins de cassis ou de l'huile de kiwi stabilisée avec les additifs antioxydants. Pour le test, on fait passer de l'air dans un tube de réaction contenant un échantillon de 5 g de matière grasse à 80°C, respectivement 90°C et l'on mesure la conductivité des produits secondaires volatils formés en cours d'oxydation et entraînés avec le courant d'air. On détermine le temps d'induction graphiquement à partir de la courbe enregistrée de la conductivité en fonction du temps par intersection de la tangente à la courbe avec l'axe des temps.

Les résultats sont indiqués dans les tableaux II et III ci-après.

Tableau II

| Huile | Additif antioxydant | Temps d'induction h) à 80°C |
|---|---|---|
| Huile de pépins de cassis | --- | 20 |
| Huile de pépins de cassis | Ascorbyl-palmitate (200 parties par million) | 40 |
| Huile de pépins de cassis | mélange antioxydant selon l'exemple 1 (1%) | 68,3 |
| Huile de pépins de cassis | mélange antioxydant selon l'exemple 1 (2%) | 78,3 |
| Huile de kiwi | --- | 5,75 |
| Huile de kiwi | Hydroxybutylanisole (600 parties par million) | 16,75 |
| Huile de kiwi | mélange antioxydant selon l'exemple 3 (1%) | 28,5 |
| Huile de kiwi | mélange antioxydant selon l'exemple 4 (1%) | 35,1 |
| Huile de kiwi | mélange antioxydant selon l'exemple 5 (1%) | 36,6 |

On constate que le mélange antioxydant selon l'invention augmente le temps d'induction de 3,4 à 3,9 fois par rapport à celui mesuré sans additif et a une activité antioxydante 1,7 à 2 fois plus importante que l'ascorbyl-palmitate dans le cas de l'huile de cassis.

Pour ce qui concerne l'huile de kiwi, le mélange contenant en sus du tocophérol augmente de 5 à 6 fois le temps d'induction par rapport à celui mesuré sans additif et a une activité antioxydante 1,7 à 2,2 fois plus importante que l'hydroxybutylanisole.

Tableau III

| Huile | Additif antioxydant | Temps d'induction (h) 90°C |
|---|---|---|
| Huile de pépins de cassis pure | --- | 8 |
| Huile de pépins de cassis pure | mélange antioxydant selon l'exemple 7 (2%) | 33 |
| Huile de pépins de cassis raffinée | --- | 10,1 |
| Huile de pépins de cassis raffinée | mélange antioxydant selon l'exemple 8 (1 %) | 24,2 |
| Huile de pépins de cassis raffinée | mélange antioxydant selon l'exemple 9 (1 %) | 17,5 |
| Huile de pépins de cassis raffinée | mélange antioxydant selon l'exemple 10 (1%) | 18,4 |

Exemples 17-20

Ces exemples concernent la préparation de compositions cosmétiques de soin de la peau contenant des lipides dont la partie lipidique est protégée contre l'oxydation par addition dans la phase lipidique du mélange oxydant liposoluble selon l'invention.

Dans ces exemples, la nomenclature utilisée est celle de la "Cosmetic, Toiletery and Flagrance Association, Inc. Washington DC" (CFTA).

Pour fabriquer les émulsions, on mélange les composants de la phase lipidique A et on la chauffe à 75°C. On prépare la phase aqueuse B et on la chauffe également à 75°C, puis on l'ajoute à la phase lipidique A en brassant lentement et on refroidit ensuite sous brassage lent jusqu'à la température ambiante, soit environ 25°C. A cette température, on ajoute lentement le cas échéant les constituants C dans l'ordre de la formule.

Pour fabriquer le produit anhydre de l'exemple 20, on mélange tous les constituants à froid, suivant l'ordre de la formule sous brassage lent.

7

17. Crème hydratante (émulsion huile-dans-l'eau)

|                                                                                    | %    |
| ---------------------------------------------------------------------------------- | ---- |
| **Phase lipidique A**                                                              | ___  |
| Peg-6-stéarate, glycerylstéarate et peg-20-cétyl éther (peg: polyéthylèneglycol)   | 15   |
| Huile de vaseline                                                                  | 5    |
| Huile de germes de blé contenant 1% du mélange antioxydant selon l'exemple 6       | 3    |
| Huiles d'amandes douces                                                            | 2    |
| Alcool cétylique                                                                   | 1    |
| Isostéaryl-isostéarate                                                             | 2    |
| 2-Octyl-docécyl-myristate                                                          | 1    |
| Cire de lanoline                                                                   | 1    |
| **Phase aqueuse B**                                                                |      |
| Méthylisothiazoline                                                                | 0,1  |
| Eau déminéralisée                                                                  | 59,6 |
| Protéine de placenta humain                                                        | 2    |
| **Additifs C**                                                                     |      |
| Propylèneglycol et extrait de calendula                                            | 2    |
| Collagène soluble (2) dans l'eau déminéralisée (4)                                 | 6    |
| Parfum                                                                             | 0,3  |

18. Crème anti-rides (émulsion huile-dans-l'eau)

|  | % |
|---|---|
| **Phase lipidique A** | |
| | |
| Cire d'abeille hydrophile non ionique | 10 |
| Huile de vaseline | 4 |
| Isostéaryl-isostéarate | 5 |
| Ethyl-linoléate | 1 |
| Huile minérale, huile de noyaux d'abricots et extrait de calendula | 4 |
| Huile d'amandes douces contenant 2% du mélange antioxydant selon l'exemple 6 | 3 |
| Huile de noyaux d'abricots | 3 |
| | |
| **Phase aqueuse B** | |
| | |
| Méthylisothiazole | 0,1 |
| Eau déminéralisée | 64,7 |
| Carbopol 934 (polymère d'acide acrylique polyréticulé) | 0,3 |
| Triéthanolamine | 0,6 |
| Propylèneglycol et extrait de sureau | 2 |
| Collagène hydrosoluble | 2 |
| Parfum | 0,3 |

19. Crème pour bébés (émulsion huile-dans-l'eau)

|  | % |
|---|---|
| **Phase lipidique A** | |
| Polyéthylèneglycol-6-32-stéarate | 10 |
| Huile d'amandes douces contenant 1% de mélange antioxydant selon l'exemple 6 | 6 |
| Huile de vaseline | 4 |
| Huile de palmiste, huile de palme et peg 6 | 3 |
| Acide stéarique | 1 |
| **Phase aqueuse B** | |
| Eau déminéralisée | 67,5 |
| Glycérine | 3 |
| Méthylisothiazoline | 1 |
| Propylèneglycol et extrait de calendula | 4 |
| Parfum | 0,5 |

20. Huile satinée (anhydre)

|  |  |
|---|---|
| Huile de tournesol contenant 1% du mélange antioxydant selon l'exemple 6 | 3 |
| Triglydérides d'acides caprylique et caprique | 30 |
| Diméthyl polysiloxane cyclique | 25,7 |
| Propylèneglycol-dipélargonate | 37,8 |
| Octyl méthoxycinnamate | 3 |
| Parfum | 0,5 |

**Revendications**

1. Mélange antioxydant liposoluble à base d'acide organique, caractérisé par le fait qu'il est constitué d'un complexe de vitamine du groupe B et d'acide organique et d'une lécithine contenant au moins 40% en poids de phosphatidylcholine.

2. Mélange selon la revendication 1, caractérisé par le fait que le complexe est l'ascorbate de niacinamide ou de pyridoxine.

3. Mélange selon la revendication 1, caractérisé par le fait qu'il contient en plus du tocophérol.

4. Mélange selon la revendication 2 ou 3, caractérisé par le fait qu'il contient un triglycéride à chaîne moyenne en C8-C10.

5. Micronutriment, caractérisé par le fait qu'il contient un mélange selon l'une des revendications 1 à 4 et des vitamines sous forme liposoluble.

6. Huile riche en acides gras insaturés protégée contre l'oxydation, caractérisée par le fait qu'elle contient un mélange selon l'une des revendications 1 à 4.

7. Produit alimentaire protégé contre l'oxydation, caractérisé par le fait qu'il contient un mélange selon l'une des revendications 1 à 4.

8. Produit cosmétique protégé contre l'oxydation, caractérisé par le fait qu'il contient un mélange selon l'une des revendications 1 à 4.

**Claims**

1. Liposoluble antioxidant mixture based on an organic acid, characterized in that it consists of a complex of a group B vitamin and an organic acid and a lecithin containing at least 40% by weight of phosphatidylcholine.

2. Mixture according to Claim 1, characterized in that the complex is niacinamide or pyridoxine ascorbate.

3. Mixture according to Claim 1, characterized in that it contains in addition tocopherol.

4. Mixture according to Claim 1 or 2, characterized in that it contains a triglyceride with an average chain length of C8 - C10.

5. Micronutrient, characterized in that it contains a mixture according to one of Claims 1 to 4 and vitamins in a liposoluble form.

6. Oil rich in unsaturated fatty acids protected against oxidation, characterized in that it contains a mixture according to one of Claims 1 to 4.

7. Food product protected against oxidation, characterized in that it contains a mixture according to one of Claims 1 to 4.

8. Cosmetic product protected against oxidation, characterized in that it contains a mixture according to one of Claims 1 to 4.

**Patentansprüche**

1. Fettlösliches Antioxidantiengemisch auf der Basis einer organischen Säure, **dadurch gekennzeichnet**, daß es von einem Komplex eines Vitamins der B-Gruppe mit einer organischen Säure und einem Lecithin, das wenigstens 40 Gew.-% Phosphatidylcholin enthält, gebildet wird.

2. Gemisch nach Anspruch 1, **dadurch gekennzeichnet**, daß der Komplex das Ascorbat von Nicotinsäureamid oder von Pyridoxin ist.

3. Gemisch nach Anspruch 1, **dadurch gekennzeichnet**, daß es zusätzlich Tocopherol enthält.

4. Gemisch nach Anspruch 2 oder 3, **dadurch gekennzeichnet**, daß es mittelkettige Triglyceride mit 8 bis 10 Kohlenstoffatomen enthält.

5. Micronährstoff, **dadurch gekennzeichnet**, daß er ein Gemisch nach einem der Ansprüche 1 bis 4 und Vitamine von der fettlöslichen Art enthält.

6. Gegen Oxidation geschütztes, an ungesättigten Fettsäuren reiches Öl, **dadurch gekennezeichnet**, daß es ein Gemisch nach einem der Ansprüche 1 bis 4 enthält.

7. Gegen Oxidation geschütztes Nahrungsmittelprodukt, **dadurch gekennzeichnet**, daß es ein Gemisch nach einem der Ansprüche 1 bis 4 enthält.

8. Gegen Oxidation geschütztes Kosmetikprodukt, **dadurch gekenzeichnet**, daß es ein Gemisch nach einem der Ansprüche 1 bis 4 enthält.